# EUROPEAN PATENT SPECIFICATION

(11) **EP 1 939 193 B1**
(45) Date of publication and mention of the grant of the patent: **10.03.2010**
(21) Application number: 07025185.5
(22) Date of filing: 28.12.2007
(51) Int. Cl.: C07D 319/06, C11B 9/00

(54) **Process for purifying acetal compounds**
Verfahren zur Reinigung von Acetalverbindungen
Processus de purification des composants d'acétal

(30) Priority: 28.12.2006 JP 2006356066
(43) Date of publication of application: 02.07.2008
(73) Proprietor: Kao Corporation, Chuo-ku Tokyo (JP)
(72) Inventor: Kotachi, Shinji, Wakayama-shi Wakayama (JP); Fukuda, Kazuyuki, Sumida-ku, Tokyo (JP)
(74) Representative: HOFFMANN EITLE

(56) References cited:
- EP-A- 0 039 029
- WO-A-99/02515
- WO-A-2006/005681
- JP-A- 2003 231 686

## Description

### FIELD OF THE INVENTION

The present invention relates to a process for purifying acetal compounds that are useful as a raw material for perfume preparations, a process for producing the acetal compounds.

### BACKGROUND OF THE INVENTION

It is known that the acetal compounds represented by the following chemical formula (IV): 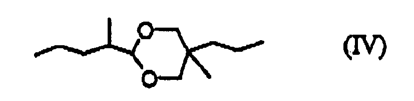 are useful as perfumes because of fragrance thereof like ligster or cashew (for example, refer to Patent Document 1). In the Patent Document 1, there is disclosed the general method for producing acetal compounds, i.e., 1,3-dioxanes in which 2-methylpentanal, 1,3-alkane diol and ethyl orthoformate are reacted with each other, and the resultant reaction product is subjected to post-treatments and then finally to vacuum distillation, and it is also described that the obtained acetal compounds have a boiling point of 72°C/0.02 mb (= 2 Pa).

Patent Document 1: JP 2-22119B

Non-Patent Document 1: "14705 Chemical Products", published by Kagaku Kogyo Nippo-sha, January 25, 2005, pp. 1218-1227

### SUMMARY OF THE INVENTION

In the industrial distillation processes, since it is not easy to achieve such a reduced pressure corresponding to a high vacuum of 2 Pa as described in the Patent Document 1, the distillation is preferably conducted under a lower vacuum. However, under such a low vacuum condition, the distillation must be conducted at a high temperature owing to increase in a boiling point of a material to be distilled. As a result, there tends to arise such a problem that the resultant product suffers from deterioration in quality owing to by-products produced by thermal decomposition thereof, thereby failing to obtain the aimed product having a good quality. Further, the industrial distillation processes generally require a longer time than the distillation described in the Patent Document 1 which is conducted in 0.1 mol scale, i.e., about 20 g scale, resulting in more remarkable heat deterioration in quality of the resultant product. On the other hand, when subjecting the acetal compounds represented by the general formula (I): 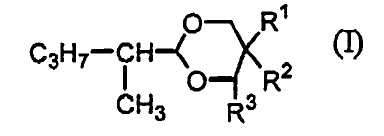 wherein R¹ is a hydrogen atom, methyl or ethyl; R² is propyl or isopropyl; and R³ is a hydrogen atom, propyl or isopropyl, to distillation under the industrially advantageous conditions, i.e., under a low vacuum and at a high temperature, there tends to arise such a problem that off-odor is generated from the acetal compound, resulting in deterioration in quality of the obtained product.

Thus, the present invention relates to a process for producing an acetal compound having a sweet floral scent in which generation of off-odor upon subjecting the acetal compound to distillation under a low vacuum and at a high temperature is suppressed, and a perfume composition containing the acetal compound.

As a result of intensive and extensive researches, the inventors have confirmed that when the acetal compound represented by the above general formula (I) such as, for example,
2-(1-methylbutyl)-5-methyl-5-propyl-1,3-dioxane is subjected to distillation under industrially advantageous conditions, i.e., under a low vacuum and at a high temperature, off-odor tends to be generated and the resultant product tends to be deteriorated in quality, and further the inventors have found that (i) the off-odor is generated by by-products produced upon the distillation such as 2,2-dimethylpentyl-2-methyl pentanoate; (ii) the by-products having a fruity green smell are important off-odor generating substances causing deterioration in quality of the acetal compound as a raw material for generally used perfume preparations; and further (iii) when limiting a content of the above by-products in the acetal compound to a specific value or lower, the product quality of the acetal compound as a raw material for perfume preparations is not adversely influenced by the by-products.

As a result of further intensive and extensive researches on the basis of the above finding, the inventors have found that when conducting the distillation in the presence of an antioxidant under a specific temperature condition, the acetal compound is obtained without deterioration in quality thereof owing to generation of off-odor therefrom, etc.

Thus, the present invention relates to a process for purifying an acetal compound represented by the general formula (I): 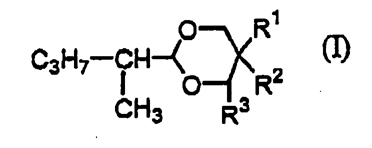 wherein R¹ is a hydrogen atom, methyl or ethyl; R² is propyl or isopropyh and R³ is a hydrogen atom, propyl or isopropyl,
which process includes the step of subjecting a raw material containing the acetal compound to distillation at a temperature of from 80 to 170°C in the presence of an antioxidant.

The present invention further relates to a process for producing an acetal compound represented by the general formula (I): 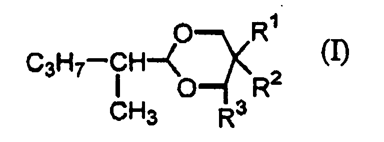 wherein R¹ is a hydrogen atom, methyl or ethyl; R² is propyl or isopropyl; and R³ is a hydrogen atom, propyl or isopropyl, which process includes the following steps (i) and (ii):
(i) reacting an aldehyde compound represented by the chemical formula (II): 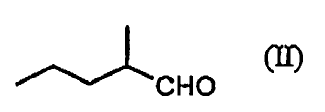 with a 1,3-diol compound represented by the general formula (III): 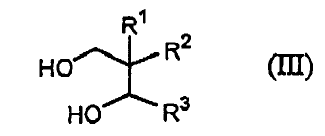 wherein R¹ is a hydrogen atom, methyl or ethyl; R² is propyl or isopropyl; and R³ is a hydrogen atom, propyl or isopropyl, to obtain a raw material containing the acetal compound; and
(ii) subjecting the raw material containing the acetal compound to distillation at a temperature of from 80 to 170°C in the presence of an antioxidant.

### EFFECT OF THE INVENTION

In the purification process and the production process according to the present invention, an acetal compound having a sweet floral scent as well as a perfume composition containing the acetal compound can be obtained without generating off-odor even upon subjecting the acetal compound to distillation under a low vacuum and at a high temperature. The acetal compound produced by the purification process and the production process according to the present invention exhibits a sweet floral scent and is useful as a raw material for perfume preparations and as an aromatizing component for aromatic agents, etc.

### DETAILED DESCRIPTION OF THE INVENTION

### [Process for Purifying Acetal Compound]

The process for purifying an acetal compound according to the present invention includes the step of subjecting a raw material containing the acetal compound represented by the general formula (I): 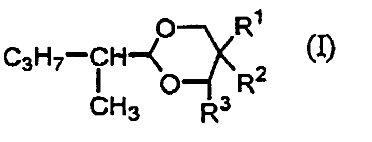 to distillation at a temperature of from 80 to 170°C in the presence of an antioxidant.

In the general formula (I), R¹ is a hydrogen atom, methyl or ethyl, R² is propyl or isopropyl, and R³ is a hydrogen atom, propyl or isopropyl. In view of obtaining a good scent, R¹ is preferably a methyl group, R² is preferably a propyl group, and R³ is preferably a hydrogen atom.

The antioxidant used in the present invention may be any known antioxidant. Examples of the antioxidant include those antioxidants described in the Non-Patent Document 1. Among these antioxidants, in view of exhibiting a good effect as aimed even when used in a small amount, preferred are phenol-based antioxidants and phosphoric acid-based antioxidants, and more preferred are phenol-based antioxidants. Examples of the phenol-based antioxidants include monophenol-based antioxidants, bisphenol-based antioxidants and polymer-type phenol-based antioxidants. Among these phenol-based antioxidants, in view of a low volatility, preferred are bisphenol-based antioxidants and polymer-type phenol-based antioxidants, and further in view of easiness of handling owing to appropriate viscosity and solubility, more preferred are bisphenol-based antioxidants.

Specific examples of the phenol-based antioxidants include monophenol-based antioxidants such as dibutylhydroxytoluene; bisphenol-based antioxidants such as 2,2'-methylene-bis(4-methyl-6-tert-butylphenol), 2,2'-methylene-bis(4-ethyl-6-tert-butylphenol) and 4,4'-butylidene-bis(3-methyl-6-tert-butylphenol); and polymer-type phenol-based antioxidants such as tetrakis-[methylene-3-(3'-5'-di-tert-butyl-4'-hyclroxyphenyl)propionate] methane and α-tocopherol. Specific examples of the phosphoric acid-based antioxidants include triphenyl phosphite, diphenyl isodecyl phosphite, 4,4'-butylidene-bis(3-methyl-6-tert-butyl-phenyl ditridecyl)phosphite, tris(nonylphenyl)phosphite, tris(mono- and/or dinonylphenyl)phosphite, diisodecyl pentaerythritol diphosphite and tris(2,4-di-tert-butylphenyl)phosphite. Among these antioxidants, preferred are bisphenol-based antioxidants such as 2,2'-methylene-bis(4-methyl-6-tert-butylphenol), 2,2'-methylene-bis(4-ethyl-6-tert-butylphenol) and 4,4'-butyhdene-bis(3-methyl-6-tert-butylphenol), α-tocopherol, and tris(2,4-di-tert-butylphenyl)phosphite. In view of a good duality of the obtained product, more preferred are 2,2'-methylene-bis(4-ethyl-6-tert-butylphenol) and α-tocophexol; and in view of easiness of handling, still more preferred is 2,2'-methylene-bis(4-ethyl-6-tert-butylphenol).

The amount of the antioxidant added is preferably from 0.001 to 50% by weight, more preferably from 0.005 to 5% by weight and still more preferably from 0.01 to 1% by weight on the basis of the raw material containing the acetal compound represented by the above general formula (I). When the amount of the antioxidant added lies within the above-specified range, deterioration in quality of the acetal compound can be suppressed, and the acetal compound can be efficiently produced.

The antioxidant may be added at any time without particular limitations as long as the antioxidant is present upon distilling the raw material containing the acetal compound according to the present invention. The antioxidant may be added before, during and/or after the reaction between the aldehyde compound and the 1,3-diol compound as described later. In view of suppressing occurrence of deterioration in quality of the obtained product owing to heat generated during the reaction, the antioxidant is preferably added before the reaction.

In the purification process of the present invention, the distillation is conducted at a temperature of from 80 to 200°C at which a vapor is condensed. In view of a good productivity and easiness of controlling pressure-reducing conditions, the distillation temperature is preferably from 80 to 170°C and more preferably from 80 to 150°C. Also, the distillation is preferably conducted under reduced pressure in order to inhibit occurrence of temperature rise in the distillation system. The distillation can be usually conducted under a vacuum of from 13 Pa to 26.7 kPa. In view of a good productivity and easiness of controlling pressure-reducing conditions, the vacuum degree used for the distillation is preferably from 27 Pa to 13.3 kPa. The temperature within the distillation vessel maintained under the above conditions is higher by usually from about 0 to about 50°C than the above vapor-condensing temperature. The distillation temperature used in the present invention is the vapor-condensing temperature.

Ordinary distillation apparatuses may be used in the above distillation procedure without any particular limitations, and the distillation procedure may be conducted using either a batch-type distillation apparatus or a continuous-type distillation apparatus. In addition, ordinary distillation columns may also be used without any particular limitations. Examples of the distillation columns include a Vigreaux column, a spinning band column, a tray column and a packed column.

The thus obtained acetal compound may contain by-products produced upon the distillation such as, for example, 2,2-dimethylpentyl-2-methyl pentanoate. The content of the by-products in the acetal compound is less than 0.5% by weight, preferably less than 0.1% by weight, more preferably less than 0.05% by weight, still more preferably less than 0.001% by weight and most preferably 0% by weight (i.e., not contained) in view of achieving a good quality of the resultant product. Therefore, the thus obtained acetal compound may be used alone or in combination with other components as an aromatizing component for perfumes, soaps, hair shampoos, rinses, body shampoos, detergents, cosmetics, spray products, aromatic agents and bath agents.

### [Process for Producing Raw Material Containing Acetal Compound]

The raw material containing the acetal compound according to the present invention can be produced by reacting an aldehyde compound represented by the chemical formula (II): 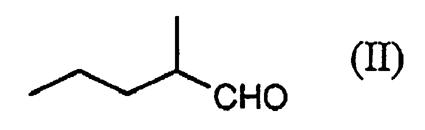 with a 1,3-diol compound represented by the general formula (III):

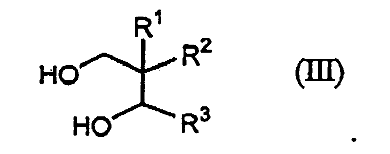

In the above general formula (III), R¹ is a hydrogen atom, methyl or ethyl; R² is propyl or isopropyh and R³ is a hydrogen atom, propyl or isopropyl. In view of obtaining a good scent, R¹ is preferably a methyl group; R² is preferably a propyl group; and R³ is preferably a hydrogen atom.

The reaction between the aldehyde compound and the 1,3-diol compound may be conducted by any known methods. For example, according to the general production procedure as described in Example 1 of JP 2-22119B, the reaction may be performed by the method represented by the following reaction formula (I):

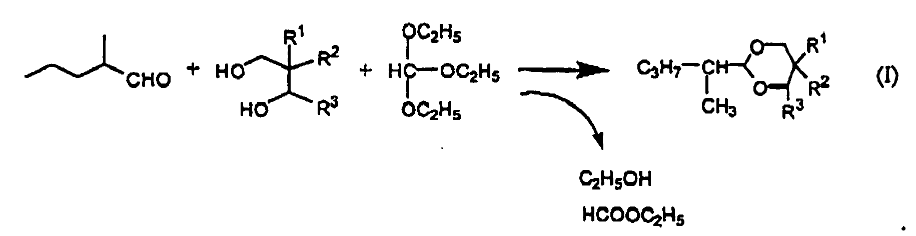

In the reaction formula (I), R¹, R² and R³ are the same as defined above. More specifically, a mixture containing the aldehyde compound and the 1,3-diol compound in equimolar amounts is mixed with an acid catalyst such as ethyl orthoformate and p-toluenesulfonic acid (and/or a monohydrate thereof), and then the obtained mixture is stirred at room temperature usually for one hour to terminate the reaction therebetween. Next, a mixture composed of ethyl formate and ethanol is gradually distilled off from the resultant reaction solution under normal pressures. The resultant cooled residue is dissolved in an ether, and the obtained solution is washed with a sodium hydroxide aqueous solution and water, dried by adding a drying agent such as sodium sulfate thereto, and then subjected to filtration and concentration, thereby obtaining the raw material containing the acetal compound which is used in the present invention.

Alternatively, the raw material containing the acetal compound which is used in the present invention may also produced by the method described in "acetalation" appearing on page 9 of JP 2002-510324A. More specifically, a mixture containing the aldehyde compound and the 1,3-diol compound in equimolar amounts is dissolved together with p-toluenesulfonic acid hydrate in a solvent such as cyclohexane, and the resultant solution is refluxed under heating to separate water as produced therefrom. After completion of the reaction, the obtained solution is cooled, washed with a saturated aqueous solution of sodium bicarbonate, and then subjected to shaking extraction using a separatory funnel shaker, etc., until the washed solution is no longer alkaline. The resultant solution is dried by adding a drying agent such as sodium sulfate thereto, and then the solvent is removed therefrom under reduced pressure using a rotary evaporator, etc., thereby obtaining the raw material used in the present invention. Further, the thus obtained raw material is purified by the above purification process to produce the acetal compound of the present invention.

Further, the raw material containing the acetal compound which is used in the present invention is preferably produced by reacting a reaction mixture essentially composed of the aldehyde compound represented by the chemical formula (II), the 1,3-diol compound represented by the general formula (III) and an acid catalyst. More specifically, in the above method, the reaction mixture may be reacted without using a solvent, a water absorbing agent, etc. Alternatively, the reaction of the reaction mixture may be conducted in the presence of a solvent, a water absorbing agent, etc., unless, for example, the reaction rate, the yield as well as the viscosity and dispersibility of the reaction mixture are adversely influenced thereby.

The molar ratio of the aldehyde compound to the 1,3-diol compound is not particularly limited, and is preferably from 1:5 to 5:1, more preferably from 1:2 to 2:1 and still more preferably from 2:3 to 3:2 in view of enhancing a productivity of the acetal compound. Although the reaction between the aldehyde compound and the 1,3-diol compound may be conducted at one time by mixing these compounds with each other, the reaction may also be conducted while adding the 1,3-diol compound to the aldehyde compound or while adding the aldehyde compound to the 1,3-diol compound in order to well control the reaction rate.

Although the aldehyde compound and the 1,3-diol compound may be reacted with each other without using any catalyst, the reaction is preferably conducted in the presence of an acid catalyst. The acid catalyst used in the above reaction is not particularly limited, and may be selected from ordinary acid catalysts. Examples of the acid catalyst usable in the reaction include phosphoric acid, sulfuric acid, nitric acid, hydrochloric acid, methanesulfonic acid, p-toluenesulfonic acid, sodium hydrogenphosphate, sodium dihydrogenphosphate, potassium phosphate, potassium hydrogenphosphate, potassium dihydrogenphosphate, potassium hydrogensulfate, sodium hydrogensulfate, copper sulfate, iron sulfate, sodium sulfate, nickel sulfate, magnesium sulfate, copper chloride, iron chloride, aluminum chloride, tin chloride, titanium chloride, iron nitrate, boron trifluoride and alumina. Among these acid catalysts, preferred are phosphoric acid, sulfuric acid, methanesulfonic acid and p-toluenesulfonic acid. The amount of the acid catalyst used is not particularly limited, and is usually from 0.01 to 20% by weight, preferably from 0.05 to 10% by weight and still more preferably from 0.1, to 5% by weight on the basis of the aldehyde compound.

The reaction between the aldehyde compound and the 1,3-diol compound is preferably conducted in the presence of an antioxidant from the viewpoint of suppressing deterioration in quality of the obtained acetal compound. Examples of the antioxidant usable in the above reaction include those compounds as described previously. The amount of the antioxidant used is preferably from 0.001 to 50% by weight, more preferably from 0.005 to 5% by weight and still more preferably from 0.01 to 1% by weight on the basis of the aldehyde compound.

In addition, the reaction between the aldehyde compound and the 1,3-diol compound may also be conducted in a solvent such as hexane, cyclohexane, heptane, benzene, toluene, xylene, diethyl ether, tetrahydrofuran, dimethyl sulfoxide and methylene chloride. As described above, the solvent may be used in such an amount that the reaction is not adversely affected even when the reaction mixture essentially composed of the aldehyde compound, the 1,3-diol compound and the acid catalyst is reacted. More specifically, the amount of the solvent used in the above reaction is preferably 1000% by weight or less, more preferably 50% by weight or less and still more preferably 1% by weight or less on the basis of the total amount of the aldehyde compound and the 1,3-diol compound, and the reaction is especially preferably conducted without using any solvent, in view of a good production efficiency.

Further, upon the reaction between the aldehyde compound and the 1,3-diol compound, an ordinary water absorbing agent, e.g., zeolites such as molecular sieves, metal salts such as sodium sulfate, calcium sulfate and magnesium sulfate, carbodiimide-based compounds such as N,N-dicyclohexyl carbodiimide and orthoesters such as methyl orthoformate and ethyl orthoformate, may also be used without any particular limitations. The amount of the water absorbing agent used is not particularly limited, and is preferably from 1 to 10000% by weight, more preferably from 5 to 1000% by weight and still more preferably from 10 to 500% by weight on the basis of the weight of the aldehyde compound.

As described above, the water absorbing agent may be used in such an amount that the reaction is not adversely affected even when the reaction mixture substantially composed of the aldehyde compound, the 1,3·diol compound and the acid catalyst is reacted. The amount of the water absorbing agent used is preferably 10% by weight or less, more preferably 5% by weight or less and still more preferably from 1% by weight or less on the basis of the weight of the aldehyde compound, and the reaction is especially preferably conducted without using any water absorbing agent.

The temperature used in the above reaction is not particularly limited, and the reaction is preferably conducted at a temperature of from -10 to 150°C, more preferably from 0 to 1.20°C and still more preferably from 30 to 100°C.

### [Perfume Composition]

The perfume composition is obtained by mixing and blending a single kind or two or more kinds of the acetal compounds represented by the above general formula (I) which are produced according to the purification process of the present invention, with other ordinary perfume components or perfume preparations having a desired composition. The amount of the acetal compound blended in the perfume composition varies depending upon kind of the perfume preparation, kind of scent as aimed, intensity of the scent, etc., and is preferably from 0.1 to 90% by weight and more preferably from 1 to 40% by weight.

Examples of the perfume components that may be used in combination with the alcohol-based compound of the present invention include natural essential oils, natural extracts or synthesized perfumes such as hydrocarbons, alcohols, phenols, esters, carbonates, aldehydes, ketones, acetals (except for the acetal compounds of the present invention), ethers, nitriles, carboxylic acids and lactones.

### EXAMPLES

### [Evaluation of Off-odor]

The off-odor generated from the acetal compounds obtained in Examples and Comparative Example was evaluated according to the following ratings.

I: No off-odor was recognized.

II: Slight off-odor was recognized, but practically usable as a floral scent compound without problems.

III: Significant off·odor was recognized, and practically unusable as a floral scent compound.

### PRODUCTION EXAMPLE 1: Production of Raw Material Containing

### 2-(1-methylbutyl)-5-methyl-5-propyl-1,3-dioxane

A 1-L flask was slowly charged with 396 g of 2-methyl-2-pxopyl-1,3-propanediol, 300 g of 2-methylpentanal and 15 g of p-toluenesulfonic acid monohydrate under a nitrogen atmosphere, and while stirring the contents of the flask, 444 g of ethyl orthoformate was dropped thereto at room temperature, followed by stirring the obtained mixture for 1 hour. The obtained reaction solution was subjected to distillation under normal pressures to remove ethanol and ethyl formate by-produced, from the solution. The obtained solution was neutralized by adding a sodium hydroxide aqueous solution and an ether thereto. After discarding a water layer of the thus neutralized solution, a remaining organic layer of the solution was washed with water, dried by adding sodium sulfate thereto, and then subjected to filtration and concentration, thereby obtaining 636 g of a raw material containing 2-(1-methylbutyl)-5-methyl-5-propyl-1,3-dioxane represented by the following chemical formula (V):

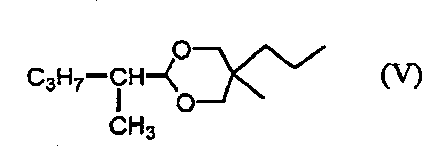

### EXAMPLE 1

Using a batch-type distillation apparatus equipped with a Vigreaux column, 100 g of the raw material containing 2-(1-methylbutyl)-5-methyl-5-propyl-1,3-dioxane as obtained in Production Example 1 was mixed with 0.1 g of 2,2'-methylene-bis(4-ethyl-6-tert-butylphenol) as an antioxidant, and the resultant mixture was subjected to distillation under such a condition that a main distillate was distilled off under a pressure of from 5 kPa to 7 kPa and at a temperature of from 138 to 143°C, thereby 97 g of a fraction composed of 2-(1-methylbutyl)-5-methyl-5-propyl-1,3-dioxane. As a result of analyzing the thus obtained fraction by gas chromatography, it was confirmed that no 2,2-dimethylpentyl-2-methyl pentanoate as a by-product was detected, and no fruity green off-odor was sensed. The purity of 2-(1-methylbuty)-5-methyl-5-propyl-1,3-dioxane in the resultant fraction as well as the results of evaluation of off-odor therefrom are shown in Table 1.

### EXAMPLE 2

The distillation procedure was conducted in the same manner as in Example 1 except for using α-tocopherol as the antioxidant, thereby obtaining a fraction. As a result of analyzing the thus obtained fraction by gas chromatography, it was confirmed that no 2,2-dimethylpentyl-2-naethyl pentanoate as a by-pxoduct was detected, and no fruity green off-odor was sensed. The purity of 2-(1-methylbutyl)-5-methyl-5-propyl-1,3-dioxane in the resultant fraction as well as the results of evaluation of off-odor therefrom are shown in Table 1.

### EXAMPLE 3

The distillation procedure was conducted in the same manner as in Example 1 except for using tris(2,4-di-text-butylphenyl)phosphite as the antioxidant, thereby obtaining a fraction. As a result of analyzing the thus obtained fraction by gas chromatography, it was confirmed that 2,2-dimethylpentyl-2-methyl pentanoate as a by-product was contained in the fraction in an amount of 0.4% by weight, and slight fruity green off·odor was sensed. The purity of 2-(1-methylbutyl)-5-methyl-5-propyl-1,3-dioxane in the resultant fraction as well as the results of evaluation of off-odor therefrom are shown in Table 1.

### COMPARATIVE EXAMPLE 1

The distillation procedure was conducted in the same manner as in Example 1 except for using no antioxidant, thereby obtaining a fraction. The purity of 2-(1-methylbutyl)-5-methyl-5-propyl-1,3-dioxane in the resultant fraction as well as the results of evaluation of off-odor therefrom are shown in Table 1.

### PRODUCTION EXAMPLE 2: Production of Raw Material Containing

### 2-(1-methylbutyl)-5-raethyl-5-propyl-1,3-dioxane

A 1-L flask was charged with 370 g of 2-methyl-2-propyl-1,3-pxopanediol, 0.6 g of 2,2'-methylene-bis(4-ethyl-6-tert-butylphenol) and 1.6 g of sulfuric acid (65% by weight) under a nitrogen atmosphere, and while heating and stirring the contents of the flask, 369 g of 2-methylpentanal was dropped thereto at a temperature of from 80 to 90°C, followed by reacting the obtained mixture at 90°C for 4 h. After completion of the reaction, the obtained reaction solution was cooled and then neutralized by adding a sodium hydroxide aqueous solution thereto. After discarding a water layer from the reaction solution, an excess amount of 2-methylpentanal was distilled off from the remaining reaction solution, thereby obtaining 549 g of a raw material containing 2-(1-methylbutyl)-5-methyl-5-propyl-1,3-dioxane represented by the above chemical formula (V).

### EXAMPLE 4

Using a batch-type distillation apparatus equipped with a distillation column packed with structured packings, 249 g of the raw material containing 2,2'-methylene-bis(4-ethyl-6-tert-butylphenol) and 2-(1-methylbutyl)-5-methyl-5-propyl-1,3-dioxane as obtained in Production Example 2 was subjected to distillation under such a condition that a main distillate was distilled off under a pressure of from 0.3 to 0.4 kPa and at a temperature of from 87 to 90°C, thereby obtaining 183 g of a fraction composed of 2-(1-methylbutyl)-5-methyl-5-pxopyl-1,3-dioxane. As a result of analyzing the thus obtained fraction by gas chromatography, it was confirmed that no 2,2-dimethylpentyl-2-methyl pentanoate as a by-product was detected, and no fruity green off-odor was sensed. The purity of 2-(1-methylbutyl)-5-methyl-5-propyl-1,3-dioxane in the resultant fraction as well as the results of evaluation of off-odor therefrom are shown in Table 1.

**TABLE 1**

| | Antioxidant | Evaluation of off-odor | Purity |
|---|---|---|---|
| Example 1 | 2,2'-methylene-bis(4-ethyl-6-tert-butylphenol) | I | 98.4% |
| Example 2 | α-tocopherol | I | 98.8% |
| Example 3 | tris(2,4-di-tert-butylphenyl)phosphite | II | 98.8% |
| Example 4 | 2,2'-methylene-bis(4-ethyl-6-tert-butylphenol) | I | 100.0% |
| Comparative Example 1 | None | III | 98.1% |

The acetal compounds obtained in Examples 1 to 3 using the antioxidant had a sweet floral scent and exhibited a very high quality. Also, the acetal compound obtained in Example 4 using the raw material obtained in Production Example 2 had a purity of 100% and no off-odor and, therefore, exhibited a very high quality. On the other hand, the acetal compound obtained in Comparative Example 1 using no antioxidant had a fruity green off-odor, and was therefore unsatisfactory as an acetal compound having a good floral scent which is applicable to raw materials for perfume preparations, etc.

### INDUSTRIAL APPLICABILITY

In accordance with the purification process and the production process of the present invention, there can be obtained an acetal compound having a sweet floral scent which is inhibited from generating off-odor even when subjecting the acetal compound to distillation under a low vacuum and at a high temperature, as well as a perfume composition containing the acetal compound. The acetal compound produced by the purification process and the production process according to the present invention exhibits a sweet floral scent and is useful as a raw material for perfume preparations and as an aromatizing component for aromatic agents, etc.

## Claims

1. A process for purifying an acetal compound represented by the general formula (I): 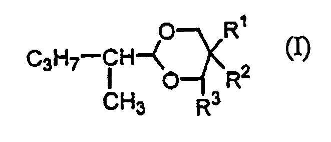 wherein R¹ is a hydrogen atom, methyl or ethyl; R² is propyl or isopropyl; and R³ is a hydrogen atom, propyl or isopropyl, the process containing the step of subjecting a raw material containing the acetal compound to distillation at a temperature of from 80 to 170°C in the presence of an antioxidant.

2. The process according to claim 1, wherein the raw material is obtained by reacting an aldehyde compound represented by the chemical formula (II): 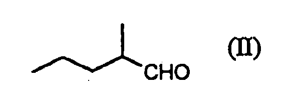 with a 1,3-diol compound represented by the general formula (III) : 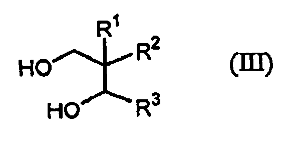 wherein R¹ is a hydrogen atom, methyl or ethyl; R² is propyl or isopropyl; and R³ is a hydrogen atom, propyl or isopropyl.

3. The process according to claim 2, wherein the antioxidant is added before the reaction, during the reaction and/or after the reaction.

4. The process according to any one of claims 1 to 3, wherein the antioxidant is a phenol-based antioxidant.

5. The process according to claim 4, wherein the phenol-based antioxidant is a bisphenol-based antioxidant.

6. The process according to any one of claims 1 to 5, wherein the antioxidant is added in an amount of from 0.001 to 50% by weight on the basis of the raw material.

7. A process for producing an acetal compound represented by the general formula (I): 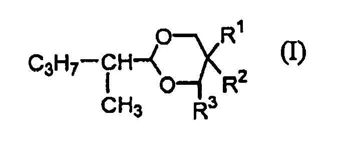 wherein R¹ is a hydrogen atom, methyl or ethyl; R² is propyl or isopropyl; and R³ is a hydrogen atom, propyl or isopropyl, the process containing the following steps (i) and (ii): (i) reacting an aldehyde compound represented by the chemical formula (II): 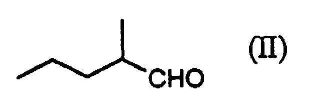 with a 1,3-diol compound represented by the general formula (III) : 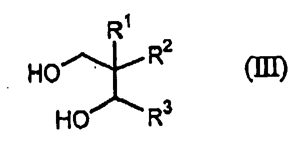 wherein R¹ is a hydrogen atom, methyl or ethyl; R² is propyl or isopropyl; and R³ is a hydrogen atom, propyl or isopropyl, to obtain a raw material containing the acetal compound; and (ii) subjecting the raw material containing the acetal compound to distillation at a temperature of from 80 to 170°C in the presence of an antioxidant.

8. The process according to claim 7, wherein the step (i) includes a step of reacting a reaction mixture essentially composed of the aldehyde compound represented by the chemical formula (II), the 1,3-diol compound represented by the general formula (III) and an acid catalyst to obtain the raw material containing the acetal compound.

## Patentansprüche

1. Verfahren zur Reinigung einer Acetalverbindung, dargestellt durch die allgemeine Formel (I): 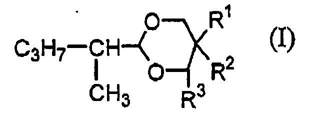 worin R¹ ein Wasserstoffatom, Methyl oder Ethyl ist; R² Propyl oder Isopropyl ist und R³ ein Wasserstoffatom, Propyl oder Isopropyl ist, wobei das Verfahren die Durchführung einer Destillation eines Ausgangsmaterials, umfassend die Acetalverbindung, bei einer Temperatur von 80 bis 170°C in der Gegenwart eines Antioxidans umfasst.

2. Verfahren nach Anspruch 1, worin das Ausgangsmaterial erhalten wird durch Reaktion einer Aldehydverbindung, dargestellt durch die chemische Formel (II): 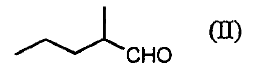 mit einer 1,3-Diolverbindung, dargestellt durch die allgemeine Formel (III) 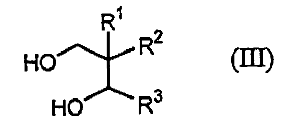 worin R¹ ein Wasserstoffatom, Methyl oder Ethyl ist, R² Propyl oder Isopropyl ist und R³ ein Wasserstoffatom, Propyl oder Isopropyl ist.

3. Verfahren nach Anspruch 2, worin das Antioxidans vor der Reaktion, während der Reaktion und/oder nach der Reaktion zugegeben wird.

4. Verfahren nach einem der Ansprüche 1 bis 3, worin das Antioxidans ein Antioxidans auf Phenolbasis ist.

5. Verfahren nach Anspruch 4, worin das Antioxidans auf Phenolbasis ein Antioxidans auf Bisphenolbasis ist.

6. Verfahren nach einem der Ansprüche 1 bis 5, worin das Antioxidans in einer Menge von 0,001 bis 50 Gew.% auf der Basis des Ausgangsmaterials zugegeben wird.

7. Verfahren zur Erzeugung einer Acetalverbindung, dargestellt durch die allgemeine Formel (I): 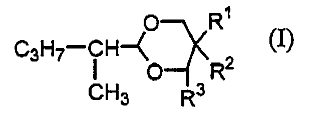 worin R¹ ein Wasserstoffatom, Methyl oder Ethyl ist; R² Propyl oder Isopropyl ist und R³ ein Wasserstoffatom, Propyl oder Isopropyl ist, wobei das Verfahren die folgenden Schritte (i) und (ii) umfasst:
(i) Reaktion einer Aldehydverbindung, dargestellt durch die chemische Formel (II): 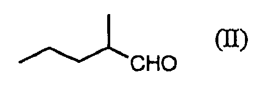 mit einer 1,3-Diolverbindung, dargestellt durch die allgemeine Formel (III): 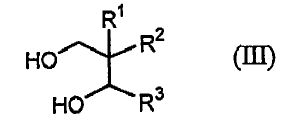 worin R¹ ein Wasserstoffatom, Methyl oder Ethyl ist, R² Propyl oder Isopropyl ist und R³ ein Wasserstoffatom, Propyl oder Isopropyl ist, unter Erhalt eines Ausgangsmaterials, umfassend die Acetalverbindung; und (ii) Durchführen einer Destillation mit dem Ausgangsmaterial, umfassend die Acetalverbindung, bei einer Temperatur von 80 bis 170°C in der Gegenwart eines Antioxidans.

8. Verfahren nach Anspruch 7, worin der Schritt (i) einen Schritt der Reaktion einer Reaktionsmischung umfasst, die sich im Wesentlichen aus der Aldehydverbindung, dargestellt durch die chemische Formel (II), der 1,3-Diolverbindung, dargestellt durch die allgemeine Formel (III), und einem sauren Katalysator zusammensetzt, unter Erhalt des Ausgangsmaterials, umfassend die Acetalverbindung.

## Revendications

1. Procédé pour purifier un composé acétal représenté par la formule générale (I) : 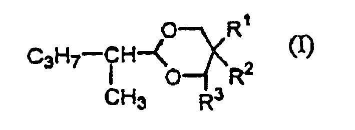 où R¹ est un atome d'hydrogène, méthyle ou éthyle ; R² est propyle ou isopropyle ; et R³ est un atome d'hydrogène, propyle ou isopropyle, le procédé contenant l'étape d'exposition d'une matière première contenant le composé acétal à une distillation à une température de 80 à 170°C en présence d'un antioxydant.

2. Procédé selon la revendication 1 où la matière première est obtenue par réaction d'un composé aldéhyde représenté par la formule chimique (II) : 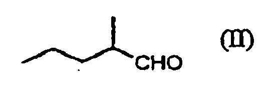 avec un composé 1,3-diol représenté par la formule générale (III) : 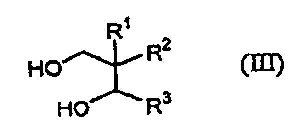 où R¹ est un atome d'hydrogène, méthyle ou éthyle ; R² est propyle ou isopropyle ; et R³ est un atome d'hydrogène, propyle ou isopropyle.

3. Procédé selon la revendication 2 où l'antioxydant est ajouté avant la réaction, pendant la réaction et/ou après la réaction.

4. Procédé selon l'une quelconque des revendications 1 à 3 où l'antioxydant est un antioxydant à base de phénol.

5. Procédé selon la revendication 4 où l'antioxydant à base de phénol est un antioxydant à base de bisphénol.

6. Procédé selon l'une quelconque des revendications 1 à 5 où l'antioxydant est ajouté en une quantité de 0,001 à 50 % en poids sur la base de la matière première.

7. Procédé pour produire un composé acétal représenté par la formule générale (I) : 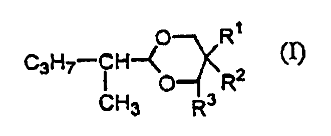 où R¹ est un atome d'hydrogène, méthyle ou éthyle ; R² est propyle ou isopropyle ; et R³ est un atome d'hydrogène, propyle ou isopropyle, le procédé contenant les étapes (i) et (ii) suivantes :
(i) réaction d'un composé aldéhyde représenté par la formule chimique (II) : 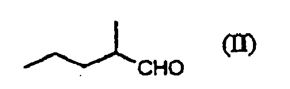 avec un composé 1,3-diol représenté par la formule générale (III) : 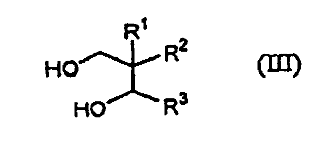 où R¹ est un atome d'hydrogène, méthyle ou éthyle ; R² est propyle ou isopropyle ; et R³ est un atome d'hydrogène, propyle ou isopropyle, pour obtenir une matière première contenant le composé acétal ; et
(ii) exposition de la matière première contenant le composé acétal à une distillation à une température de 80 à 170°C en présence d'un antioxydant.

8. Procédé selon la revendication 7 où l'étape (i) inclut une étape de réaction d'un mélange réactionnel composé essentiellement du composé aldéhyde représenté par la formule chimique (II), du composé 1,3-diol représenté par la formule générale (III) et d'un catalyseur acide pour obtenir la matière première contenant le composé acétal.
